# EUROPÄISCHE PATENTSCHRIFT

(11) **EP 1 613 579 B1**
(45) Veröffentlichungstag und Bekanntmachung des Hinweises auf die Patenterteilung: **28.07.2010**
(21) Anmeldenummer: 04722177.5
(22) Anmeldetag: 20.03.2004
(51) Int. Cl.: C07C 45/35

(54) **VERFAHREN DER HETEROGEN KATALYSIERTEN PARTIELLEN GASPHASENOXIDATION VON PROPEN ZU ACROLEIN**
METHOD FOR THE HETEROGENEOUSLY CATALYSED PARTIAL GAS PHASE OXIDATION OF PROPENE TO FORM ACROLEIN
PROCEDE D'OXYDATION PARTIELLE EN PHASE GAZEUSE DE PROPENE EN ACROLEINE PAR CATALYSE HETEROGENE

(30) Priorität: 25.03.2003 DE 10313212; 06.06.2003 US 476162 P
(43) Veröffentlichungstag der Anmeldung: 11.01.2006
(73) Patentinhaber: BASF SE, 67056 Ludwigshafen (DE)
(72) Erfinder: DIETERLE, Martin, 68167 Mannheim (DE); PETZOLDT, Jochen, 68163 Mannheim (DE); MÜLLER-ENGEL, Klaus, Joachim, 76297 Stutensee (DE)
(86) Internationale Anmeldenummer: PCT/EP2004/002935
(87) Internationale Veröffentlichungsnummer: WO 2004/085363

(56) Entgegenhaltungen:
- DE-A- 19 948 241

## Beschreibung

Vorliegende Erfindung betrifft ein Verfahren der heterogen katalysierten partiellen Gasphasenoxidation von Propen zu Acrolein, bei dem man ein Propen, molekularen Sauerstoff und wenigstens ein Inertgas enthaltendes Reaktionsgasausgangsgemisch, das den molekularen Sauerstoff und das Propen in einem molaren Verhältnis O₂:C₃H₆ ≥ 1 enthält, in einer Reaktionsstufe so über eine Festbettkatalysatorschüttung, die in zwei räumlich aufeinander folgenden Reaktionszonen A,B angeordnet ist, wobei die Temperatur der Reaktionszone A eine Temperatur im Bereich von 290 bis 380°C und die Temperatur der Reaktionszone B ebenfalls eine Temperatur im Bereich von 290 bis 380°C ist, und deren Aktivmasse wenigstens ein die Elemente Mo, Fe und Bi enthaltendes Multimetalloxid ist, führt, dass sich die Reaktionszone A bis zu einem Umsatz des Propens von 40 bis 80 mol-% erstreckt und bei einmaligem Durchgang des Reaktionsgasausgangsgemischs durch die gesamte Festbettkatalysatorschüttung der Propenumsatz ≥ 90 mol-% und die Selektivität der Acoleinbildung, bezogen auf umgesetztes Propen, ≥ 90 mol-% beträgt, wobei die zeitliche Abfolge, in der das Reaktionsgasausgangsgemisch die Reaktionszonen durchströmt, der alphabetischen Abfolge der Reaktionszonen entspricht.

Das vorgenannte Verfahren der katalytischen Gasphasenoxidation von Propen zu Acrolein ist allgemein bekannt (vgl. z.B. DE-A 19910506) und insbesondere als erste Oxidationsstufe bei der Herstellung von Acrylsäure durch zweistufige katalytische Gasphasenoxidation ausgehend von Propen von Bedeutung. Acrylsäure ist ein bedeutendes Monomeres, das als solches oder in Form eines Alkylesters zur Erzeugung von z.B. als Klebstoffen geeigneten Polymerisaten Verwendung findet. Acrolein ist ein bedeutendes Zwischenprodukt.

Neben molekularem Sauerstoff und den Reaktanden enthält das Reaktionsgasausgangsgemisch u.a. deshalb Inertgas, um das Reaktionsgas außerhalb des Explosionsbereiches zu halten.

Eine Zielsetzung einer solchen heterogen katalysierten partiellen Gasphasenoxidation von Propen zu Acrolein besteht darin, bei einmaligem Durchgang des Reaktionsgasgemisches durch die Reaktionsstufe unter ansonsten vorgegebenen Randbedingungen eine möglichst hohe Ausbeute A^{AC} an Acrolein (Hauptprodukt) zu erzielen (das ist die Molzahl von zu Acrolein umgesetztem Propen, bezogen auf die Molzahl an eingesetztem Propen).

Eine weitere Zielsetzung einer solchen heterogen katalysierten partiellen Gasphasenoxidation von Propen zu Acrolein besteht darin, eine möglichst hohe Raum-Zeit-Ausbeute (RZA^{AC}) an Acrolein zu erzielen (das ist bei einer kontinuierlichen Verfahrensweise die je Stunde und Volumen der verwendeten Festbettkatalysatorschüttung in Litern erzeugte Gesamtmenge an Acrolein).

Bei gleich bleibender gegebener Ausbeute A^{AC} ist die Raum-Zeit-Ausbeute um so größer, je größer die Belastung der Festbettkatalysatorschüttung der Reaktionsstufe mit Propen ist (darunter wird die Menge an Propen in Normlitern (= NL; das Volumen in Litern, das die entsprechende Propenmenge bei Normalbedingungen, d.h. bei 25°C und 1 bar, einnehmen würde) verstanden, die als Bestandteil des Reaktionsgasausgangsgemisches pro Stunde durch einen Liter an Festbettkatalysatorschüttung geführt wird).

Die Lehren der Schriften WO 01/36364, DE-A 19927624, DE-A 19948248, DE-A 19948523, DE-A 19948241 und DE-A 19910506 sind daher darauf gerichtet, bei im wesentlichen gleich bleibender A^{AC} die Propenbelastung der Festbettkatalysatorschüttung der Reaktionsstufe mit Propen signifikant zu erhöhen. Dies gelingt im wesentlichen dadurch, dass die Festbettkatalysatorschüttung in der Reaktionsstufe in jeweils zwei räumlich aufeinander folgenden Temperaturzonen (Reaktionszonen) angeordnet wird. Die Propenbelastung der Festbettkätalysatorschüttung wird dabei zu ≥ 160 Nl/l Festbettkatalysatorschüttung-h gewählt und die Temperatur der jeweils zweiten (in Strömungsrichtung des Reaktionsgasgemisches) Temperaturzone muss wenigstens 5°C oberhalb der Temperatur der ersten Temperaturzone liegen.

In ähnlicher Weise lehrt auch die EP-A 1106598 ein Verfahren der Hochlastfahrweise für die heterogen katalysierte partielle Gasphasenoxidation von Propen zu Acrolein, bei dem die Festbettkatalysatorschüttung der Reaktionsstufe in mehreren Temperaturzonen angeordnet ist. Dabei kann der Temperaturunterschied einer in Strömungsrichtung des Reaktionsgasgemisches nachfolgenden Temperaturzone gemäß der Lehre der EP-A 1106598 sowohl mehr als auch weniger als 5°C oberhalb der Temperatur der vorhergehenden Temperaturzone liegen, wobei es die EP-A 1106598 völlig offen lässt, unter welchen Bedingungen ein größerer und unter welchen Bedingungen ein kleinerer Temperaturunterschied angewendet werden sollte.

Auch lässt die EP-A 1106598 völlig offen, was unter der Temperatur einer Reaktions- bzw. Temperaturzone zu verstehen ist.

Im Unterschied dazu wird in den übrigen Schriften des zitierten Standes der Technik unter der Temperatur einer Reaktionszone die Temperatur der in der Reaktionszone befindlichen Festbettkatalysatorschüttung bei Ausübung des Verfahrens in Abwesenheit einer chemischen Reaktion verstanden. Ist diese Temperatur innerhalb der Reaktionszone nicht konstant, so meint der Begriff Temperatur einer Reaktionszone hier den (Zahlen)mittelwert der Temperatur der Festbettkatalysatorschüttung längs der Reaktionszone. Wesentlich ist dabei, dass die Temperierung der einzelnen Reaktionszonen im wesentlichen unabhängig voneinander erfolgt, weshalb einer Reaktionszone stets eine Temperaturzone entspricht. Vorstehende Definition der Temperatur einer Reaktionszone soll auch in dieser Schrift gelten.

Da die heterogen katalysierte partielle Gasphasenoxidation von Propen zu Acrolein eine ausgeprägt exotherme Reaktion ist, ist die Temperatur des Reaktionsgasgemisches beim reaktiven Durchgang durch die Festbettkatalysatorschüttung in der Regel von der Temperatur einer Reaktionszone verschieden. Sie liegt normalerweise oberhalb der Temperatur der Reaktionszone und durchläuft innerhalb einer Reaktionszone in der Regel ein Maximum (Heißpunktmaximum) oder fällt von einem Maximalwert ausgehend ab.

Nachteilig an den Lehren des Standes der Technik ist jedoch, dass sie ausschließlich darauf gerichtet sind, eine Mehrzonenanordnung unter hoher Propenlast zu betreiben. Dies ist insofern nachteilig, als mit einer solchen Verfahrensweise unabdingbar eine hohe RZA^{AC} einhergeht. Diese setzt aber einen entsprechenden Marktbedarf an Acrolein und/oder Acrylsäure voraus. Ist letzterer nicht gegeben (z.B. temporär), muss die Mehrzonenanordnung in notwendiger Weise bei geringeren Propenlasten betrieben werden, wobei dann außerdem eine möglichst hohe Selektivität der Acroleinbildung, bezogen auf umgesetztes Propen, als anzustrebende Zielgröße im Vordergrund steht (S^{AC}). Das ist die bei einmaligem Durchgang durch die Mehrzonenanordnung gebildete molare Menge an Acrolein bezogen auf die Molzahl an dabei umgesetztem Propen.

Die Aufgabe der vorliegende Erfindung bestand daher darin, ein Verfahren der heterogen katalysierten partiellen Gasphasenoxidation von Propen zu Acrolein in einer Mehrzonenanordnung zur Verfügung zu stellen, bei dem bei Propenlasten von < 160 NI/l-h die Acroleinbildung mit möglichst hoher Selektivität erfolgt.

Demgemäß wurde ein Verfahren der heterogen katalysierten partiellen Gasphasenoxidation von Propen zu Acrolein, bei dem man ein Propen , molekularen Sauerstoff und wenigstens ein Inertgas enthaltendes Reaktionsgasausgangsgemisch, das den molekularen Sauerstoff und das Propen in einem molaren Verhältnis O₂:C₃H₆ ≥ 1 enthält, in einer Reaktionsstufe so über eine Festbettkatalysatorschüttung, die in zwei räumlich aufeinander folgenden Reaktionszonen A,B angeordnet ist, wobei die Temperatur der Reaktionszone A eine Temperatur im Bereich von 290 bis 380°C und die Temperatur der Reaktionszone B ebenfalls eine Temperatur im Bereich von 290 bis 380°C ist, und deren Aktivmasse wenigstens ein die Elemente Mo, Fe und Bi enthaltendes Multimetalloxid ist, führt, dass sich die Reaktionszone A bis zu einem Umsatz des Propens von 40 bis 80 mol-% erstreckt und bei einmaligem Durchgang des Reaktionsgasausgangsgemischs durch die gesamte Festbettkatalysatorschüttung der Propenumsatz ≥ 90 mol-% und die Selektivität der Acroleinbildung, bezogen auf umgesetztes Propen, ≥ 90 mol-% beträgt, wobei die zeitliche Abfolge, in der das Reaktionsgasausgangsgemisch die Reaktionszonen durchströmt, der alphabetischen Abfolge der Reaktionszonen entspricht, gefunden, das dadurch gekennzeichnet ist, dass
a) die Belastung der Festbettkatalysatorschüttung mit dem im Reaktionsgasausgangsgemisch enthaltenen Propen < 160 Nl Propen/l Festbettkatalysatorschüttung-h und ≥ 90 Nl Propen/l Festbettkatalysatorschüttung^{.}h beträgt,
b) die volumenspezifische Aktivität der Festbettkatalysatorschüttung in Strömungsrichtung des Reaktionsgasgemisches über die Festbettkatalysatorschüttung entweder konstant ist oder wenigstens einmal zunimmt; und
c) die Differenz T^{maxA} - T^{maxB}, gebildet aus der höchsten Temperatur T^{maxA}, die das Reaktionsgasgemisch innerhalb der Reaktionszone A aufweist, und der höchsten Temperatur T^{maxB}, die das Reaktionsgasgemisch innerhalb der Reaktionszone B aufweist, > 0°C beträgt.

Vorzugsweise nimmt die volumenspezifische Aktivität der Festbettkatalysatorschüttung in Strömungsrichtung wenigstens einmal zu.

In der Regel wird beim erfindungsgemäßen Verfahren die Differenz T^{maxA} - T^{maxB} nicht mehr als 80°C betragen. Erfindungsgemäß bevorzugt beträgt T^{maxA} - T^{maxB} ≥ 3°C und ≤ 70°C. Ganz besonders bevorzugt beträgt T^{maxA} - T^{maxB} beim erfindungsgemäßen Verfahren ≥ 20°C und ≤ 60°C.

Das erfindungsgemäße Verfahren erweist sich z.B. als vorteilhaft, wenn die Belastung der Festbettkatalysatorschüttung mit dem im Reaktionsgasausgangsgemisch enthaltenen Propen ≥ 90 Nl Propen /l^{.}h und ≤ 155 Nl Propen /l^{.}h, oder ≥ 100 Nl Propen /l^{.}h und ≤ 150 Nl Propen /l^{.}h, oder ≥ 110 Nl Propen /l^{.}h und ≤ 145 Nl Propen /l^{.}h, oder ≥ 120 Nl Propen /l^{.}h und ≤ 140 Nl Propen /l^{.}h, oder ≥ 125 Nl Propen /l^{.}h und ≤ 135 Nl Propen /l^{.}h beträgt.

Selbstverständlich kann das erfindungsgemäße Verfahren auch dann angewendet werden, wenn die Belastung der Festbettkatalysatorschüttung mit dem im Reaktionsgasgemisch enthaltenen Acrolein < 90 NI Acrolein/l·h beträgt. Der Betrieb einer Mehrzonenanordnung bei solchermaßen geringen Reaktandenlasten wäre jedoch kaum noch wirtschaftlich.

Die erfindungsgemäß geforderten Differenzen T^{maxA}- T^{maxB} stellen sich bei der Ausübung des erfindungsgemäßen Verfahrens normalerweise dann ein, wenn einerseits sowohl die Temperatur der Reaktionszone A als auch die Temperatur der Reaktionszone B im Bereich von 290 bis 380°C liegt und andererseits die Differenz zwischen der Temperatur der Reaktionszone B (T_{B}) und der Temperatur der Reaktionszone A (T_{A}), d.h., T_{B}-T_{A}, ≤ 0°C und ≥ -20°C oder ≥ -10°C, bzw. ≤ 0°C und ≥ -5°C, oder häufig ≤ 0°C und ≥ -3°C beträgt.

D.h., im Gegensatz zur Lehre des Standes der Technik für hohe Lasten, wird beim erfindungsgemäßen Verfahren die Temperatur der Nachfolgezone normalerweise geringer sein als die Temperatur der vorausgehenden Reaktionszone.

Die vorgenannte Aussage betreffend die Temperaturdifferenzen T_{B}-T_{A} gilt auch dann, wenn die Temperatur der Reaktionszone A im bevorzugten Bereich von 305 bis 365°C bzw. im besonders bevorzugten Bereich von 310 bis 340°C liegt.

Der Arbeitsdruck kann beim erfindungsgemäßen Verfahren sowohl unterhalb von Normaldruck (z.B. bis zu 0,5 bar) als auch oberhalb von Normaldruck liegen. Typischerweise wird der Arbeitsdruck bei Werten von 1 bis 5 bar, häufig 1 bis 3 bar liegen. Normalerweise wird der Reaktionsdruck 100 bar nicht überschreiten.

Erfindungsgemäß bevorzugt erstreckt sich die Reaktionszone A bis zu einem Umsatz des Propens von 50 bis 70 mol-% bzw. 60 bis 70 mol-%.

In der Regel kann der auf den einfachen Durchgang bezogene Propenumsatz beim erfindungsgemäßen Verfahren ≥ 92 mol-%, oder ≥ 94 mol-%, oder ≥ 96 mol-% betragen. Die Selektivität der Acroleinbildung wird dabei regelmäßig ≥ 92 mol-%, bzw. ≥ 94 mol-%, häufig ≥ 95 mol-% oder ≥ 96 mol-% bzw. ≥ 97 mol-% betragen.

Das molare Verhältnis von O₂:Propen im Reaktionsgasausgangsgemisch muß erfindungsgemäß ≥ 1 betragen. Häufig liegt es bei Werten > 1. Üblicherweise wird dieses Verhältnis bei Werten ≤ 3 liegen. Häufig beträgt das molare Verhältnis von O₂:Propen im Reaktionsgasausgangsgemisch erfindungsgemäß 1 bis 2 bzw. 1 bis 1,5.

Als Quelle für den erforderlichen molekularen Sauerstoff kommt sowohl Luft, als auch an molekularem Stickstoff entreicherte Luft in Betracht.

Als Katalysatoren für die Festbettkatalysatorschüttung des erfindungsgemäßen Verfahrens kommen alle diejenigen in Betracht, deren Aktivmasse wenigstens ein Mo, Bi und Fe enthaltendes Multimetalloxid ist.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für die Festbettkatalysatorschüttung die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Ox • 10 SiO₂ aufweist. Ferner sind hervorzuheben das Beispiel mit der laufenden Nr. 3 aus der DE-A 19855913 (Stöchiometrie: Mo₁₂Co₇Fe₃Bi_{0,6}K_{0,08}Si_{1,6}Ox) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 10101695 bzw. WO 02/062737.

Weiterhin sind das Beispiel 1 aus der DE-A 10046957 (Stöchiometrie: [Bi₂W₂O₉ x 2WO₃]_{0,5} • [Mo₁₂Co_{5,6}Fe_{2,94}Si_{1,59}K_{0,08}Oₓ]₁) als Hohlzylinder(Ring)vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: Mo₁₂Bi_{1,0}Fe₃Co₇Si_{1,6}K_{0,08}), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für die Katalysatoren der Festbettkatalysatorschüttung geeigneten Multimetalloxidaktivmassen lässt sich unter der allgemeinen Formel I

Mo₁₂BiₐFe_{b}X¹_{c}X²_{d}X³ₑX⁴_{f}Oₙ (I),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,

a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozeß vorzugsweise durch Sprühtrocknung der wässrigen Mischung mit Austrittstemperaturen von 100 bis 150°C erfolgt.

Üblicherweise werden die Multimetalloxidaktivmassen der allgemeinen Formel I in der Festbettkatalysatorschüttung nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven O-xidmassen wird selbstredend an die gewünschte Schalendicke angepasst (vgl. EP-A 714 700).

Für die Katalysatoren der Reaktionsstufe geeignete Multimetalloxidaktivmassen sind ferner Massen der allgemeinen Formel II

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (II),

in der die Variablen folgende Bedeutung haben:
Y¹ = nur Wismut oder Wismut und wenigstens eines der Elemente Tellur, Antimon, Zinn und Kupfer,
Y² = Molybdän oder Molybdän und Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶ = Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in II bestimmt werden und
p,q = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäße Multimetalloxidmassen II sind solche, in denen Y¹ nur Wismut ist.

Unter diesen werden wiederum jene bevorzugt, die der allgemeinen Formel III

[Bi_{a"}Z²_{b"}O_{x"}]_{p"} [Z²₁₂Z³_{c"}Z⁴_{d"}Fe_{e"}Z⁵_{f"}Z⁶_{g"}Z⁷_{h"}O_{y"}]_{q"} (III)

in der die Varianten folgende Bedeutung haben:
Z² = Molybdän oder Molybdän und Wolfram,
Z³ = Nickel und/oder Kobalt,
Z⁴ = Thallium, ein Alkalimetall und/oder ein Erdalkalimetall,
Z⁵ = Phosphor, Arsen, Bor, Antimon, Zinn, Cer und/oder Blei,
Z⁶ = Silicium, Aluminium, Titan und/oder Zirkonium,
Z⁷ = Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in III bestimmt werden,
p",q"=Zahlen, deren Verhältnis p"/q" 0,1 bis 5, vorzugsweise 0,5 bis 2 beträgt,
entsprechen, wobei diejenigen Massen III ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}) vorliegen, deren Größtdurchmesser im Bereich 1 nm bis 100 µm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913 beschrieben.

Zur Bereitung der Festbettkatalysatorschüttung können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden, Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungskörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die Festbettkatalysatorschüttung nicht verändert. D.h., die für einen einzelnen Katalysatorformkörper verwendete Aktivmasse kann zwar ein Gemisch aus verschiedenen die Elemente Mo, Fe und Bi enthaltenden Multimetalloxiden sein, für alle Katalysatorformkörper der Festbettkatalysatorschüttung ist dann jedoch das gleiche Gemisch zu verwenden.

Die volumenspezifische (d.h., die auf die Einheit des Volumens normierte) Aktivität kann in diesem Fall z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen der Schüttung enthaltene Aktivmasse bzw. Katalysatoraktivität.

Eine in Strömungsrichtung des Reaktionsgasgemisches über die Festbettkatalysatorschüttung wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünhungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Lässt man den Anteil an Verdünnungsformkörpern konstant oder werden überhaupt keine Verdünnungsformkörper in der Festbettkatalysatorschüttung mitverwendet, resultiert dann eine konstante volumenspezifische Aktivität in Strömungsrichtung des Reaktionsgasgemisches über die Festbettkatalysatorschüttung. Eine Zunahme der volumenspezifischen Aktivität ist aber auch z.B. dadurch möglich, dass man bei gleichbleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für die Festbettkatalysatorschüttung aber auch Mischungen aus Katalysatoren mit chemisch unterschiedlicher Aktivmassenzusammensetzung und als folge dieser verschiedenen Zusammensetzung unterschiedlicher Aktivität verwendet werden. Diese Mischungen können wiederum mit inerten Verdünnungskörpern verdünnt werden.

Im Normalfall wird beim erfindungsgemäßen Verfahren innerhalb der Festbettkatalysatorschüttung in Strömungsrichtung des Reaktionsgasgemisches die volumenspezifische Aktivität niemals abnehmen.

Vorab und/oder im Anschluss an die Festbettkatalysatorschüttung können sich ausschließlich aus Inertmaterial (z.B. nur Verdünnungsformkörpern) bestehende Schüttungen befinden (sie werden in dieser Schrift begrifflich nicht der Festbettkatalysatorschüttung zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Erfindungsgemäß bevorzugt ist beim erfindungsgemäßen Verfahren die Festbettkatalysatorschüftung in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung befindet sich dann erfindungsgemäß vorteilhaft bis zum Ende der Länge der Festbettkatalysatorschüttung (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

In anwendungstechnisch zweckmäßiger Weise erfolgt die Durchführung der erfindungsgemäßen Reaktionsstufe des erfindungsgemäßen Verfahrens in einem Zweizonenrohrbündelreaktor, wie er z.B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für eine Durchführung der ersten Reaktionsstufe des erfindungsgemäßen Verfahrens geeignet.

D.h., in einfachster Weise befindet sich die erfindungsgemäß zu verwendende Festbettkatalysatorschüttung (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Reaktionszone. D.h., in einfachster Weise umströmt z.B. ein Salzbad A denjenigen Abschnitt der Rohre (die Reaktionszone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Reaktionszone B), in welchem sich die oxidative Anschlussumsetzung des Propens (beim einfachen Durchgang) bis zum Erreichen eines Umsatzwertes von wenigstens 90 mol-% vollzieht (bei Bedarf können sich an die erfindungsgemäß anzuwendenden Reaktionszonen A,B weitere Reaktionszonen anschließen, die auf individuellen Temperaturen gehalten werden).

Anwendungstechnisch zweckmäßig umfasst die Reaktionsstufe des erfindungsgemäßen Verfahrens keine weiteren Reaktionszonen. D.h., das Salzbad B umströmt zweckmäßig den Abschnitt der Rohre, in welchem sich die oxidative Anschlussumsetzung des Propens (beim einfachen Durchgang) bis zu einem Umsatzwert ≥ 90 mol-%, oder ≥ 92 mol-% oder ≥ 94 mol-% oder mehr vollzieht.

Üblicherweise liegt der Beginn der Reaktionszone B hinter dem Heißpunktmaximum der Reaktionszone A.

Die beiden Salzbäder A,B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Reaktionszone A eine Gleichströmung und in der Reaktionszone B eine Gegenströmung (oder umgekehrt) angewandt werden.

Selbstverständlich kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird beim erfindungsgemäßen Verfahren das Reaktionsgasausgangsgemisch der Festbettkatalysatorschüttung auf die Reaktionstemperatur vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, dass die Temperatur des Wärmeaustauschmittels von der Eintrittsstelle in die Reaktionszone bis zur Austrittstelle aus der Reaktionszone (bedingt durch die Exothermie der Reaktion) um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Reaktionszone A liegt erfindungsgemäß normalerweise im Bereich von 290 bis 380°C, bevorzugt im Bereich von 305 bis 365°C und besonders bevorzugt im Bereich von 310 bis 340°C bzw. bei 330°C. Die Eintrittstemperatur des Wärmeaustauschmittels in die Reaktionszone B liegt erfindungsgemäß normalerweise ebenfalls im Bereich von 290 bis 380°C, gleichzeitig aber normalerweise ≥0°C bis ≤20°C oder bis ≤10°C, bzw. ≤0°C und ≤5°C, oder häufig ≥0°C und ≤3°C unterhalb der Eintrittstemperatur des in die Reaktionszone A eintretenden Wärmeaustauschmittels.

Es sei an dieser Stelle auch noch einmal darauf hingewiesen, dass für eine Durchführung der Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone B eine Teilmenge an die Reaktionszone A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb einer individuellen Reaktionszone wie in der EP-A 382098 beschrieben gestaltet werden.

Aus dem die Reaktionsstufe verlassenden Produktgasgemisch kann das Acrolein in an sich bekannter Weise abgetrennt und das dabei verbleibende Restgas in an sich bekannter Weise wenigstens teilweise als Verdünnungsgas in die Propenpartialoxidation rückgeführt werden. In zweckmäßiger Weise wird man es jedoch zur Beschickung einer nachfolgenden heterogen katalysierten Partialoxidation des darin enthaltenen Acroleins zu Acrylsäure verwenden. Zeckmäßigerweise wird man es vor dem Eintritt in eine solche nachfolgende Reaktionsstufe auf direkte und/oder indirekte Weise abkühlen, um so eine Nachvollverbrennung von Teilen des in der Propenoxidationsstufe gebildeten Acroleins zu unterdrücken.

Üblicherweise wird dazu zwischen die beiden Reaktionsstufen ein Nachkühler geschaltet. Dies kann im einfachsten Fall ein indirekter Rohrbündelwärmeträger sein. Anschließend wird meist noch Sauerstoff in Form von Luft ergänzt, um den für eine solche Acroleinoxidation vorteilhaften überstöchiometrischen Sauerstoffgehalt zur Verfügung zu stellen.

Der Propenanteil im Reaktionsgasausgangsgemisch kann beim erfindungsgemäßen Verfahren z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das erfindungsgemäße Verfahren bei einem Propen : Sauerstoff : indifferente Gase (einschließlich Wasserdampf) Volumenverhältnis im Reaktionsgasausgangsgemisch 1 von 1 : (1,0 bis 3,0) : (5 bis 25), vorzugsweise 1 : (1,7 bis 2,3) : (10 bis 15) durchführen. Im Regelfall wird das indifferente (inerte) Gas zu wenigstens 20 % seines Volumens aus molekularem Stickstoff bestehen. Es kann aber auch zu ≥30 Vol.-%, oder zu ≥40 Vol.-%, oder zu ≥50 Vol.-%, oder zu ≥60 Vol.-%, oder zu ≥70 Vol.-%, oder zur ≥80 Vol.-%, oder zu ≥90 Vol.-%, oder zu ≥95 Vol.-% aus molekularem Stickstoff bestehen (generell sollen in dieser Schrift inerte Verdünnungsgase solche sein, die sich beim einmaligen Durchgang durch die Reaktionsstufe zu weniger als 5 %, bevorzugt zu weniger als 2 % umsetzen; das sind neben molekularem Stickstoff z.B. Gase wie Propan, Ethan, Methan, Pentan, Butan, CO₂, CO, Wasserdampf und/oder Edelgase). Natürlich kann das inerte Verdünnungsgas beim erfindungsgemäßen Verfahren auch zu bis zu 50 mol-%, bzw. bis zu 75 mol-% und mehr aus Propan bestehen. Bestandteil des Verdünnungsgases kann auch Kreisgas sein, wie es nach der Abtrennung des Acroleins und/oder der Acrylsäure (z.B. im Fall einer nachgeschalteten zweiten Oxidationsstufe) aus dem Produktgasgemisch verbleibt.

Erfindungsgemäß günstige Reaktionsgasausgangsgemische sind z.B. auch solche, die aus

| | |
|---|---|
| 6 bis 15 (bevorzugt 7 bis 11) Vol-% | Propen, |
| 4 bis 20 (bevorzugt 6 bis 12) Vol.-% | Wasser, |
| ≥0 bis 10 (bevorzugt ≥0 bis 5) Vol.-% | von Propen, Wasser Sauerstoff und Stick-stoff verschiedenen Bestandteilen, |

soviel molekularem Sauerstoff, dass das molare Verhältnis von enthaltenem molekularem Sauerstoff zu enthaltenem Propen 1,5 bis 2,5 (bevorzugt 1,6 bis 2,2) beträgt, und als Restmenge bis zu 100 Vol.-% Gesamtmenge aus molekularem Stickstoff zusammengesetzt sind,
wie sie die DE-A 10302715 empfiehlt.

An dieser Stelle sei noch festgehalten, dass als Aktivmassen für die Festbettkatalysatorschüttung auch die Multimetalloxidmassen der DE-A 10261186 günstig sind.

Generell lässt sich die volumenspezifische Aktivität zwischen zwei Festbettkatalysatorschüttungszonen experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleiche Propen enthaltende Reaktionsgasausgangsgemisch geführt wird. Die höhere umgesetzte Menge an Propen weist die höhere volumenspezifische Aktivität aus.

Erfindungsgemäß günstige Ausgestaltungen eines Zweizonenrohrbündelreaktors für die erfindungsgemäße Reaktionsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

| Kontaktrohre: | |
|---|---|
| Material der Kontaktrohre: | ferritischer Stahl; |
| Abmessungen der Kontaktrohre: | z.B. 3500 mm Länge; |
| | z.B. 30 mm Außendurchmesser; |
| | z.B. 2 mm Wandstärke; |

Anzahl der Kontaktrohre im Rohrbündel: z.B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z.B. der selben Länge und Wandstärke, aber mit einem Außendurchmesser von z.B. 33,4 mm und einer zentrierten Thermohülse von z.B. 8 mm Außendurchmesser und z.B. 1 mm Wandstärke;

Reaktor (Material wie die Kontaktrohre):
Zylinderförmiger Behälter eines Innendurchmessers von 6000 - 8000 mm;
Reaktorhauben plattiert mit Edelstahl des Typs 1,4541; Plattierungsdicke: einige mm;
ringförmig angeordnetes Rohrbündel, z.B. mit einem freien zentralen Raum;
Durchmesser des zentralen freien Raumes: z.B. 1000 - 2500 mm (z.B. 1200 mm, oder 1400 mm, oder 1600 mm, oder 1800 mm, oder 2000 mm, oder 2200 mm, oder 2400 mm);
normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z.B. 36 mm, oder 38 mm, oder 40 mm, oder 42 mm, oder 44 mm;
die Kontaktrohre sind mit ihren Enden in Kontaktrohrböden (oberer Boden und unterer Boden z.B. mit einer Dicke von 100 - 200 mm) abdichtend befestigt und münden am oberen Ende in eine mit dem Behälter verbundene Haube, die einen Zulaß für das Reaktionsgasausgangsgemisch aufweist; ein z.B. auf der halben Kontaktrohrlänge befindliches Trennblech einer Dicke von 20 -100 mm, teilt den Reaktorraum symmetrisch in zwei Reaktionszonen (Temperaturzonen) A (obere Zone) und B (untere Zone); jede Reaktionszone wird durch eine Umlenkscheibe in 2 äquidistante Längsabschnitte geteilt;
die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;
jede Zone wird durch eine eigene Salzpumpe mit Salzschmelze als Wärmeträger versorgt; die Zufuhr der Salzschmelze ist z.B. unterhalb der Umlenkscheibe und die Entnahme ist z.B. oberhalb der Umlenkscheibe;
aus beiden Salzschmelzekreisen wird z.B. ein Teilstrom entnommen und z.B. in einem gemeinsamen oder zwei getrennten indirekten Wärmetauschern abgekühlt (Dampferzeugung);
im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;
durch im Reaktormantel befindliche Fenster gelangt die Salzschmelze zum Rohrbündel; die Einströmung erfolgt z.B. in radialer Richtung zum Rohrbündel;
die Salzschmelze fließt in jeder Zone der Vorgabe des Umlenkblechs folgend z.B. in der Abfolge
   - von außen nach innen,
   - von innen nach außen,
   um die Kontaktrohre;
durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;
über jede Reaktionszone wird die Salzschmelze von unten nach oben geführt;

Das Reaktionsgasgemisch verlässt den Reaktor der erfindungsgemäßen Reaktionsstufe mit einer Temperatur wenige Grad höher als die Salzbadeintrittstemperatur. Das Reaktionsgasgemisch wird für die Weiterverarbeitung in einer nachfolgenden Acroleinoxidationsstufe zweckmäßigerweise in einem separaten Nachkühler, der dem Reaktor der 1. Stufe nachgeschaltet ist, auf 220°C bis 280°C, bevorzugt 240°C bis 260°C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

Salzschmelze:
Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 m³/h betragen.

### Stromführung:

das Reaktionsgasausgangsgemisch strömt zweckmäßig von oben nach unten durch den Erststufenreaktor, während die unterschiedlich temperierten Salzschmelzen der einzelnen Zonen zweckmäßig von unten nach oben gefördert werden;

Kontaktrohr- und Thermorohrbeschickung (von oben nach unten) z.B.:
- Abschnitt 1: 50 cm Länge: Steatitringe der Geometrie 7 mm x 7 mm x 4 mm (Außendurchmesser x Länge x Innendurchmesser) als Vorschüttung.
- Abschnitt 2: 140 cm Länge: Katalysatorbeschickung mit einem homogenen Gemisch aus 30 Gew.-*% an Steatitringen der Geometrie 5 mm x 3 mm x 2 mm (Außendurchmesser x Länge x Innendurchmesser) und 70 Gew.-% Vollkatalysator aus Abschnitt 3.
- Abschnitt 3: 160 cm Länge: Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2 WO₃]_{0,5} [Mo₁₂Co_{5,5}Fe_{2,94}Si_{1,59}K_{0,08}Oₓ]₁).

Alternativ kann die Kontaktrohr- und Thermorohrbeschickung (von oben nach unten) auch so aussehen:
- Abschnitt 1: 50 cm Länge: Steatitringe der Geometrie 7 mm x 7 mm x 4 mm (Außendurchmesser x Länge x Innendurchmesser) als Vorschüttung.
- Abschnitt 2: 300 cm Länge: Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} • [Mo₁₂Co_{5,6}Fe_{2,94}Si_{1,94}K_{0,08}Oₓ]₁).

In allen genannten Beschickungen kann der Vollkatalysator aus Beispiel 1 der DE-A 10046957 auch ersetzt werden durch:
a) einen Katalysator gemäß Beispiel 1c aus der EP-A 15565 oder einen gemäß diesem Beispiel herzustellenden Katalysator, der jedoch die Aktivmasse Mo₁₂Ni_{6,5}Zn₂Fe₂Bi₁P_{0,0065}K_{0,06}Oₓ • 10 SiO₂ aufweist;
b) Beispiel Nr. 3 aus der DE-A 19855913 als Hohlzylindervollkatalysator der Geometrie 5 mm x 3 mm x 2 mm bzw. 5 mm x 2 mm x 2 mm;
c) Multimetalloxid II - Vollkatalysator gemäß Beispie 1 der DE-A 19746210;
d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht.

Im Übrigen wird die Festbettkatalysatorschüttung erfindungsgemäß zweckmäßig so gewählt (z.B. durch Verdünnung mit z.B. Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80°C nicht überschreitet. Meist beträgt dieser Temperaturunterschied ≤70°C, häufig liegt er bei 20 bis 70°C, vorzugsweise ist dieser Temperaturunterschied gering. Außerdem wird die Festbettkatalysatorschüttung aus Sicherheitsgründen in dem Fachmann an sich bekannter Weise so gewählt (z.B. durch Verdünnung mit z.B. Inertmaterial), dass die "peak-to-salt-temperature sensitivity" gemäß Definition in der EP-A 1106598 ≤ 9°C, oder ≤ 7°C, oder ≤ 5°C, oder ≤3°C beträgt.

Die beschriebene Reaktoranordnung einschließlich ihrer Beschickungen mit Festbettkatalysator kann auch bei hohen Propenlasten wie in den Schriften WO 01/36364, DE-A 19927624, DE-A 19948248, DE-A 19948523, DE-A 19948241, DE-A 19910506, DE-A 10302715 und EP-A 1106598 beschrieben betrieben werden.

Bevorzugt können die Reaktionszonen A und B dabei die in dieser Schrift empfohlenen Temperaturen aufweisen, jedoch so, dass die jeweils zweite Reaktionszone, gemäß der Lehre der vorgenannten Schriften, eine höhere Temperatur als die jeweils erste Reaktionszone aufweist. Die Heißpunkttemperatur in der zweiten Reaktionszone liegt vorzugsweise stets unterhalb derjenigen der jeweils ersten Reaktionszone.

Bei den erfindungsgemäßen Propenlasten resultiert jedoch mit der erfindungsgemäßen Verfahrensweise eine, relativ zu der für hohe Propenlasten empfohlenen Verfahrensweise, erhöhte Selektivität der Acroleinbildung.

### Beispiele und Vergleichsbeispiele

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:
- Abschnitt 1: 50 cm Länge: Steatitringe der Geometrie 7 mm x 7 mm x 4 mm (Außendurchmesser x Länge x Innendurchmesser) als Vorschüttung.
- Abschnitt 2: 140 cm Länge: Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.
- Abschnitt 3: 160 cm Länge: Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} [Mo₁₂Co_{5,5}Fe_{2,94}Si_{1,59}K_{0,08}Oₓ]₁).

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

Die vorstehend beschriebene erste Reaktionsstufe wird mit einem Reaktionsgasausgangsgemisch 1 der nachfolgenden Zusammensetzung kontinuierlich beschickt, wobei die Belastung und die Thermostatisierung des ersten Reaktionsrohres variiert werden:
6 bis 6,5 Vol.-% Propen,
3 bis 3,5 Vol.-% H₂O,
0,3 bis 0,5 Vol.-% CO,
0,8 bis 1,2 Vol.-% CO₂,
0,01 bis 0,04 Vol.-% Acrolein,
10,4 bis 10,7 Vol.-% O₂ und
als Restmenge ad 100 % molekularer Stickstoff.

Das Reaktionsgasgemisch durchströmt das Reaktionsrohr von oben nach unten.

Der Druck am Eingang des Reaktionsrohres variiert in Abhängigkeit von der gewählten Propenbelastung zwischen 2,3 und 3,1 bar.

Dem Produktgasgemisch wird am Reaktionsrohrausgang eine kleine Probe für die gaschromatographische Analyse genommen. Am Ende der Reaktionszone A befindet sich ebenfalls eine Analysenstelle.

Die Ergebnisse in Abhängigkeit von Belastungen und Salzbadtemperaturen zeigt die nachfolgende Tabelle (der Buchstabe B in Klammern bedeutet Beispiel und der Buchstabe V in Klammern bedeutet Vergleichsbeispiel).
- T_{A}, T_{B},: stehen für die Temperaturen der umgepumpten Salzbäder in den Reaktionszonen A und B.
- U_{PA}: ist der Propenumsatz am Ende der Reaktionszone A in mol-%.
- U_{PB}: ist der Propenumsatz am Ende der Reaktionszone B in mol-%.
- S_{AC}: ist die Selektivität der Acroleinbildung im Produktgasgemisch bezogen auf umgesetztes Propen in mol-%.
- S_{AA}: ist die Selektivität der Acrylsäurenebenproduktbildung im Produktgasgemisch bezogen auf umgesetztes Propen in mol-%.
- T^{maxA}, T^{maxB}: stehen für die höchste Temperatur des Reaktionsgasgemisches innerhalb der Reaktionszonen A und B in °C.

**Tabelle**

| Propenbelastung (NI/l·h) | T_{A} | T_{B} | T^{maxA} | T^{maxB} | U_{PA} | U_{PB} | S_{AC} | S_{AA} |
|---|---|---|---|---|---|---|---|---|
| 130 (B) | 319 | 319 | 384 | 351 | 66,7 | 95,1 | 92,6 | 5,1 |
| 130 (B) | 327 | 313 | 400 | 330 | 70,3 | 94,8 | 93,9 | 4,4 |
| 130 (V) | 311 | 325 | 361 | 372 | 60,8 | 95,0 | 90,9 | 6,2 |
| 185 (V) | 322 | 336 | 380 | 368 | 64,5 | 94,9 | 90,6 | 7,4 |
| 185 (V) | 310 | 344 | 353 | 383 | 57,2 | 95,2 | 89,2 | 8,1 |

## Patentansprüche

1. Ein Verfahren der heterogen katalysierten partiellen Gasphasenoxidation von Propen zu Acrolein, bei dem man ein Propen , molekularen Sauerstoff und wenigstens ein Inertgas enthaltendes Reaktionsgasausgangsgemisch, das den molekularen Sauerstoff und das Propen in einem molaren Verhältnis O_{2:}C₃H₆ ≥ 1 enthält, in einer Reaktionsstufe so über eine Festbettkatalysatorschüttung, die in zwei räumlich aufeinander folgenden Reaktionszonen A,B angeordnet ist, wobei die Temperatur der Reaktionszone A eine Temperatur im Bereich von 290 bis 380°C und die Temperatur der Reaktionszone B ebenfalls eine Temperatur im Bereich von 290 bis 380°C ist, und deren Aktivmasse wenigstens ein die Elemente Mo, Fe und Bi enthaltendes Multimetalloxid ist, führt, dass sich die Reaktionszone A bis zu einem Umsatz des Propens von 40 bis 80 mol-% erstreckt und bei einmaligem Durchgang des Reaktionsgasausgangsgemischs durch die gesamte Festbettkatalysatorschüttung der Propenumsatz ≥ 90 mol-% und die Selektivität der Acroleinbildung, bezogen auf umgesetztes Propen, ≥ 90 mol-% beträgt, wobei die zeitliche Abfolge, in der das Reaktionsgasausgangsgemisch die Reaktionszonen durchströmt, der alphabetischen Abfolge der Reaktionszonen entspricht, das **dadurch gekennzeichnet ist, dass**
a) die Belastung der Festbettkatalysatorschüttung mit dem im Reaktionsgasausgangsgemisch enthaltenen Propen < 160 Nl Propen/l Festbettkatalysatorschüttung^{.}h und ≥ 90 Nl Propen/l Festbettkatalysatorschüttung^{.}h beträgt,
b) die volumenspezifische Aktivität der Festbettkatalysatorschüttung in Strömungsrichtung des Reaktionsgasgemisches über die Festbettkatalysatorschüttung entweder konstant ist oder wenigstens einmal zunimmt, und
c) die Differenz T^{maxA} - T^{maxB}, gebildet aus der höchsten Temperatur T^{maxA}, die das Reaktionsgasgemisch innerhalb der Reaktionszone A aufweist, und der höchsten Temperatur T^{maxB}, die das Reaktionsgasgemisch innerhalb der Reaktionszone B aufweist, > 0°C beträgt.

2. Verfahren nach Anspruch 1, **dadurch gekennzeichnet, dass** die Differenz T^{maxA}-T^{maxB} > 0°C und ≤80°C beträgt.

3. Verfahren nach Anspruch 1, **dadurch gekennzeichnet, dass** die Differenz T^{maxA}-T^{maxB} ≥3°C und ≤70°C beträgt.

4. Verfahren nach Anspruch 1, **dadurch gekennzeichnet, dass** die Differenz T^{maxA} - T^{maxB} ≥20°C und ≤60°C beträgt.

5. Verfahren nach einem der Ansprüche 1 bis 4, **dadurch gekennzeichnet, dass** die Belastung der Festbettkatalysatorschüttung mit dem im Reaktionsgasausgangsgemisch enthaltenen Propen ≥90 Nl Propen/l·h und ≤ 155 Nl Propen/l·h beträgt.

6. Verfahren nach einem der Ansprüche 1 bis 4, **dadurch gekennzeichnet, dass** die Belastung der Festbettkatalysatorschüttung mit dem im Reaktionsgasausgangsgemisch enthaltenen Propen ≥100 Nl Propen/l·h und ≤ 150 Nl Propen/l·h beträgt.

7. Verfahren nach einem der Ansprüche 1 bis 6, **dadurch gekennzeichnet, dass** die Aktivmasse der Festbettkatalysatorschüttung wenigstens eine Multimetalloxidmasse der allgemeinen Formel I
Mo₁₂BiₐFe_{b}X¹_{c}X²_{d}X³ₑX⁴_{f}Oₙ (I),
in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,
a = 0,5 bis 5,
b = 0,01 bis 5, vorzugsweise 2 bis 4,
c = 0 bis 10; vorzugsweise 3 bis 10,
d = 0 bis 2, vorzugsweise 0,02 bis 2, '
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
ist.

8. Verfahren nach einem der Ansprüche 1 bis 7, **dadurch gekennzeichnet, dass** die volumenspezifische Aktivität der Festbettkatalysatorschüttung wenigstens einmal zunimmt.

## Claims

1. A process for partially oxidizing propene to acrolein in the gas phase under heterogeneous catalysis by conducting a starting reaction gas mixture comprising propene, molecular oxygen and at least one inert gas, and comprising the molecular oxygen and the propene in a molar O₂:C₃H₆ ratio of ≥ 1, in one reaction stage over a fixed catalyst bed which is arranged in two spatially successive reaction zones A, B, the temperature of reaction zone A being a temperature in the range from 290 to 380°C and the temperature of reaction zone B likewise being a temperature in the range from 290 to 380°C, and whose active composition is at least one multimetal oxide comprising the elements Mo, Fe and Bi, in such a way that reaction zone A extends up to a conversion of propene of from 40 to 80 mol% and, on single pass of the starting reaction gas mixture through the entire fixed catalyst bed, the propene conversion is ≥ 90 mol% and the selectivity of acrolein formation, based on converted propene, is > 90 mol%, the chronological sequence in which the starting reaction gas mixture flows through the reaction zones corresponding to the alphabetic sequence of the reaction zones, wherein
a) the hourly space velocity of the propene comprised in the starting reaction gas mixture on the fixed catalyst bed is < 160 1 (STP) of propene/l of fixed catalyst bed·≥h and ≥ 90 l (STP) of propene/l of fixed catalyst bed·h,
b) the volume-specific activity of the fixed catalyst bed is either constant or increases at least once in the flow direction of the reaction gas mixture over the fixed catalyst bed, and
c) the difference T^{maxA} - T^{maxB}, formed from the highest temperature T^{maxA} which the reaction gas mixture has within reaction zone A and the highest temperature T^{maxB} which the reaction gas mixture has within reaction zone B, is > 0°C.

2. The process according to claim 1, wherein the difference T^{maxA} - T^{maxB} is > 0°C and ≤ 80°C.

3. The process according to claim 1, wherein the difference T^{maxA} - T^{maxB} is > 3°C and ≤ 70°C.

4. The process according to claim 1, wherein the difference T^{maxA} - T^{maxB} is ≥ 20°C and ≤ 60°C.

5. The process according to any of claims 1 to 4, wherein the hourly space velocity of the propene comprised in the starting reaction gas mixture on the fixed catalyst bed is ≥ 90 l (STP) of propene/l·h and ≤ 155 l (STP) of propene/l·h.

6. The process according to any of claims 1 to 4, wherein the hourly space velocity of the propene comprised in the starting reaction gas mixture on the fixed catalyst bed is ≥ 100 l (STP) of propene/l·h and ≤ 150 l (STP) of propene/l·h.

7. The process according to any of claims 1 to 6, wherein the active composition of the fixed catalyst bed is at least one multimetal oxide of the general formula I
Mo₁₂BiₐFe_{b}X¹_{c}X²_{d}X³ₑX⁴_{f}Oₙ (I)
where the variables are defined as follows:
X¹= nickel and/or cobalt,
X²= thallium, an alkali metal and/or an alkaline earth metal,
X³= zinc, phosphorus, arsenic, boron, antimony, tin, cerium, lead and/or tungsten,
X⁴= silicon, aluminum, titanium and/or zirconium,
a = from 0.5 to 5,
b = from 0.01 to 5, preferably from 2 to 4,
c = from 0 to 10, preferably from 3 to 10,
d = from 0 to 2, preferably from 0.02 to 2,
e = from 0 to 8, preferably from 0 to 5,
f = from 0 to 10 and
n = a number which is determined by the valency and frequency of the elements other than oxygen in I.

8. The process according to any of claims 1 to 7, wherein the volume-specific activity of the fixed catalyst bed increases at least once.

## Revendications

1. Procédé d'oxydation partielle en phase gazeuse sous catalyse hétérogène de propène en acroléine, selon lequel un mélange gazeux réactionnel initial contenant du propène, de l'oxygène moléculaire et au moins un gaz inerte, qui contient l'oxygène moléculaire et le propène en un rapport molaire O₂:C₃H₆ ≥ 1, est passé lors d'une étape de réaction sur un garnissage catalytique en lit fixe, qui est placé dans deux zones de réaction A, B successives dans l'espace, la température de la zone de réaction A étant une température dans la plage allant de 290 à 380 °C et la température dans la zone de réaction B étant également une température dans la plage allant de 290 à 380 °C, et dont la masse active est au moins un oxyde de plusieurs métaux contenant les éléments Mo, Fe et Bi, de manière à ce que la zone de réaction A s'étende jusqu'à une conversion du propène de 40 à 80 % en moles et à ce que lors d'un passage unique du mélange gazeux réactionnel initial au travers de l'ensemble du garnissage catalytique en lit fixe, la conversion du propène soit ≥ 90 % en moles et la sélectivité de la formation de l'acroléine, par rapport au propène réagi, soit ≥ 90 % en moles, l'ordre chronologique selon lequel le mélange gazeux réactionnel initial traverse les zones de réaction correspondant à l'ordre alphabétique des zones de réaction, **caractérisé en ce que**
a) le chargement du garnissage catalytique en lit fixe avec le propène contenu dans le mélange gazeux réactionnel initial est < 160 Nl de propène/l de garnissage catalytique en lit fixe · h et ≥ 90 Nl de propène/l de garnissage catalytique en lit fixe · h,
b) l'activité spécifique au volume du garnissage catalytique en lit fixe dans la direction de l'écoulement du mélange gazeux réactionnel sur le garnissage catalytique en lit fixe est constante ou augmente au moins une fois, et
c) la différence T^{maxA} - T^{maxB}, formée à partir de la température la plus élevée T^{maxA} que le mélange gazeux réactionnel présente dans la zone de réaction A et la température la plus élevée T^{maxB} que le mélange gazeux réactionnel présente dans la zone de réaction B, est > 0 °C.

2. Procédé selon la revendication 1, **caractérisé en ce que** la différence T^{maxA} - T^{maxB} est > 0 °C et ≤ 80 °C.

3. Procédé selon la revendication 1, **caractérisé en ce que** la différence T^{maxA} - T^{maxB} est ≥ 3 °C et ≤ 70 °C.

4. Procédé selon la revendication 1, **caractérisé en ce que** la différence T^{maxA} - T^{maxB} est ≥ 20 °C et ≤ 60 °C.

5. Procédé selon l'une quelconque des revendications 1 à 4, **caractérisé en ce que** le chargement du garnissage catalytique en lit fixe avec le propène contenu dans le mélange gazeux réactionnel initial est ≥ 90 Nl de propène/l · h et ≤ 155 Nl de propène/l · h.

6. Procédé selon l'une quelconque des revendications 1 à 4, **caractérisé en ce que** le chargement du garnissage catalytique en lit fixe avec le propène contenu dans le mélange gazeux réactionnel initial est ≥ 100 Nl de propène/l · h et ≤ 150 Nl de propène/l · h.

7. Procédé selon l'une quelconque des revendications 1 à 6, **caractérisé en ce que** la masse active du garnissage catalytique en lit fixe est au moins une masse d'oxyde de plusieurs métaux de formule générale I
Mo₁₂BiₐFe_{b}X¹_{c}X²_{d}X³ₑX⁴_{f}Oₙ (I) ,
dans laquelle les variables présentent la signification suivante :
X¹ = nickel et/ou cobalt,
X² = thallium, un métal alcalin et/ou un métal alcalino-terreux,
X³ = zinc, phosphore, arsenic, bore, antimoine, étain, cérium, plomb et/ou tungstène,
X⁴ = silicium, aluminium, titane et/ou zirconium,
a = 0, 5 à 5,
b = 0,01 à 5, de préférence 2 à 4,
c = 0 à 10, de préférence 3 à 10,
d = 0 à 2, de préférence 0,02 à 2,
e = 0 à 8, de préférence 0 à 5,
f = 0 à 10 et
n = un nombre qui est déterminé par la valence et la fréquence des éléments différents de l'oxygène dans I.

8. Procédé selon l'une quelconque des revendications 1 à 7, **caractérisé en ce que** l'activité spécifique au volume du garnissage catalytique en lit fixe augmente au moins une fois.
